# EUROPÄISCHE PATENTSCHRIFT

(11) **EP 2 406 000 B1**
(45) Veröffentlichungstag und Bekanntmachung des Hinweises auf die Patenterteilung: **19.09.2012**
(21) Anmeldenummer: 10714568.2
(22) Anmeldetag: 01.03.2010
(51) Int. Cl.: B01J 8/06, C07C 5/42

(54) **VERFAHREN UND VORRICHTUNG ZUR GLEICHMÄSSIGEN DAMPFERZEUGUNG AUS DER ABWÄRME EINER ALKANDEHYDRIERUNG**
METHOD AND APPARATUS FOR A CONSTANT STEAM GENERATION FROM THE WASTE HEAT OF AN ALKANE DEHYDROGENATION
PROCÉDÉ ET DISPOSITIF POUR GÉNÉRER UNIFORMÉMENT DE LA VAPEUR À PARTIR DE LA CHALEUR DÉGAGÉE PAR UNE DÉSHYDROGÉNATION D'ALCANES

(30) Priorität: 13.03.2009 DE 102009012663
(43) Veröffentlichungstag der Anmeldung: 18.01.2012
(73) Patentinhaber: ThyssenKrupp Uhde GmbH, 44141 Dortmund (DE)
(72) Erfinder: HEINRITZ-ADRIAN, Max, 48155 Münster (DE); WENZEL, Sascha, 44791 Bochum (DE)
(74) Vertreter: Fleuchaus, Andrea
(86) Internationale Anmeldenummer: PCT/EP2010/001238
(87) Internationale Veröffentlichungsnummer: WO 2010/102734

(56) Entgegenhaltungen:
- EP-A2- 0 179 322
- EP-A2- 1 419 992
- US-A1- 2003 190 503

## Beschreibung

Die Erfindung betrifft einen oder mehrere Brenner zur zusätzlichen Beheizung im Rauchgaskanal eines Reaktors zur Durchführung einer endothermen Reaktion, durch die sich eine annähernd konstante Menge an Rauchgas produzieren lässt, wobei der Reaktor einen Dampferzeuger enthält, der sich im Ausgang des Rauchgaskanals des Heizraumes befindet, und wobei diese Brenner als Hilfsbrenner zum Ausgleich der Rauchgasreduzierung im Rauchgaskanal des Heizraumes genutzt werden, die üblicherweise während der Regenerationsphase der exothermen Regenerierung des verwendeten Katalysators auftritt. Die Erfindung betrifft auch eine Vorrichtung, die einen oder mehrere Hilfsbrenner umfasst, die sich in einem Reaktor zur Durchführung einer endothermen Reaktion mit den dazu notwendigen Einrichtungen befindet und mit dem sich die Menge an Rauchgas im Ausgang des Rauchgaskanals, in dem sich ein Dampferzeuger befindet, steuern lässt. Auf diese Weise wird der dem Dampferzeuger entnehmbare Dampfstrom erheblich vergleichmäßigt, was ein Vorteil beim Betrieb von Turbinen oder Kompressoren ist. Besonders geeignet ist diese Vorrichtung für Reaktoren, in denen typischerweise Alkandehydrierungen durchgeführt werden.

Ein gängiges Verfahren zur Durchführung von Alkandehydrierungen ist die Überleitung eines alkanhaltigen Kohlenwasserstoffgemisches über einen Dehydrierungskatalysator, durch den eine Reaktion des im Gasgemisch enthaltenen Alkanes in das korrespondierende Alken ermöglicht wird. Der Katalysator befindet sich in einer typischen Ausführungsform in abwärtsgerichteten Reaktionsrohren, durch die das Reaktionsgas über einen Eingangskanal einströmt, so dass das Produktgas, das als Komponente das erwünschte Alken enthält, am Ausgang der Reaktionsrohre entnommen werden kann. Die Reaktion ist endotherm, wodurch die Reaktionsrohre von außen beheizt werden müssen. Dies geschieht üblicherweise mittels eines Reaktors, der einen Heizraum enthält, durch den die Reaktionsrohre geführt werden, und der durch ein Heizgas beheizbar ist. Die Reaktionsrohre sind dabei gegenüber dem Heizraum abgeschlossen. Der Heizraum mündet in einen Rauchgaskanal, in dem das heiße Rauchgas thermisch genutzt und schließlich in einen Kamin abgeführt wird.

Eine typische Ausführungsform für das Verfahren zur Alkandehydrierung mit einer entsprechenden Vorrichtung beschreibt die WO 2004/039920 A2. Eine Auswahl an verschiedenen Dehydrierungsverfahren und den dabei verwendeten Katalysatoren findet man in der Veröffentlichungsschrift F. Buonomo, D. Sonfillipo, F. Trifirö, Handbook of Heterogeneous Catalysis, 1st Edition, VCH, Weinheim, 1997 p. 2140 ff. und den darin zitierten Literaturstellen.

Das Rauchgas aus der Beheizung wird aus dem Heizraum durch Rauchgaskanäle ausgeführt. Dabei besitzt es je nach Ausführungsform eine Temperatur von etwa 1000°C. Um die Wärme aus der Beheizung der Reaktionsrohre weiter zu nutzen, befindet sich am oder hinter dem Ausgang des Rauchgaskanals für das Rauchgas typischerweise ein Dampferzeuger.

Eine Alkandehydrierung läuft typischerweise unter Bildung von kohlenstoffhaltigen Nebenprodukten ab, die sich nach einer gewissen Reaktionszeit auf dem Katalysator ablagern. Dadurch sinkt der Umsatz der Reaktion und die Produktion an erwünschtem Alken. Aus diesem Grund wird die Reaktion nach einer gewissen Zeit unterbrochen und die Überleitung von Reaktionsgas über den Katalysator gestoppt. Daran anschließend wird in einer typischen Ausführungsform ein sauerstoffhaltiges Gas über den Katalysator geleitet. Dadurch werden die kohlenstoffhaltigen Beläge abgetragen und der Katalysator wird regeneriert. Nach der Regeneration wird die Alkandehydrierung in dem betreffenden Reaktionsrohr oder Reaktor erneut durchgeführt. Das Verfahren wird nach dieser Verfahrensweise zyklisch durchgeführt.

Da die Dehydrierung von Alkanen endotherm und die Regeneration des Katalysators exotherm ist, muss dem Reaktor während der Regeneration erheblich weniger Wärme zugeführt werden als während des Normalbetriebes. Üblicherweise werden dazu die Brenner während der Regenerationsphase mit weniger Heizgas betrieben, so dass auch weniger Rauchgas entsteht.

Die WO 2007/118825 A1 beschreibt ein Verfahren zur Herstellung von Olefinen aus Kohlenwasserstoffen und eine Vorrichtung zur Durchführung des Verfahrens. Durch das Ausschalten der Brenner während der Regenerationsphase wird die Wärmezufuhr in das Katalysatorbett unterbrochen, so dass bei der Regeneration durch Überleiten eines sauerstoffhaltigen Gases keine Wärme mehr in das Katalysatorbett gelangt und der Katalysator vor Überhitzung und Beschädigung geschützt wird. Die Brenner besitzen zur Ausführung des Verfahrens eine Vorrichtung zum Abschalten und werden zum Wiederanfahren nach der Regeneration mit Pilotbrennern wieder gezündet. Sowohl die Heizbrenner als auch die Pilotbrenner können mit einer Überwachungseinrichtung ausgestattet sein. Es werden keinerlei Hinweise zur Erzeugung von Dampf durch die Verwendung eines Dampferzeugers und einer Kompensation der Heizunterbrechung gegeben.

Die EP 179322 B1 beschreibt ein Verfahren zum exothermen Regenerieren eines Katalysators, der während einer endothermen katalytischen Umsetzung innerhalb eines diskontinuierlichen Verfahrens deaktiviert worden ist. Durch eine Reduzierung der Brennerleistung auf weniger als 50 Prozent der Ursprungsleistung, vorzugsweise weniger als 10 Prozent der ursprünglichen Brennerleistung, was durch eine Verringerung der Zufuhr an Heizmittel durchgeführt wird, wird eine Einsparung an Heizmedium und Verbrennungsluft erreicht. Als in Frage kommende Prozesse zur Anwendung werden insbesondere die Dehydrierung von i-Butan, n-Butan oder Gemische daraus genannt. Bei Verwendung mehrerer Reaktoren ist es möglich, diese im Wechsel zu betreiben, so dass insgesamt keine Änderung der zugeführten Heizmittel, der Verbrennungsströme und kein Lastwechsel im Abhitzesystem erfolgt. Auch diese Lehre gibt keinerlei Hinweise zur Erzeugung von Dampf durch die Verwendung eines Dampferzeugers oder zur Kompensation der Heizunterbrechung.

Bei der Drosselung des Heizprozesses für die Regenerierung des Katalysators kommt es zu einer Reduzierung des Rauchgaszuflusses in die Dampferzeuger, die im Rauchgaskanal angeordnet ist. Dies ist problematisch, denn der durch die Dampferzeuger erzeugte Dampf wird in einer wichtigen Ausführungsform zum Antrieb eines Kompressors mittels einer Dampfturbine genutzt. Der Dehydrierprozess arbeitet so, dass sowohl während des Normalbetriebes als auch während der Regeneration Dampf zur Verwendung als Turbinendampf zugeführt wird. Während der Regenerationsphase ist die Dampfeigenproduktion geringer. Gleichzeitig ist der Dampfverbrauch im Regerationsbetrieb jedoch ähnlich hoch wie im Normalbetrieb. Daher bestimmt die zugeführte Dampfmenge während der Regenerationsphase die Menge des zugeführten Dampfes für die Dehydrierungsanlage.

Es besteht deshalb die Aufgabe, ein Verfahren zur endotherm katalysierten Herstellung eines Alkens bereitzustellen, die eine während der Regenerationsphase möglichst hohe Menge an Rauchgas zum Betrieb eines Dampferzeugers bereitstellt, so dass eine konstante Menge an Dampf über den gesamten Zyklus "Produktion-Regeneration" bereitgestellt werden kann. Die Erfindung soll auch eine Vorrichtung hierzu bereitstellen. Diese soll es auch ermöglichen, eine Überwachung und eine Fernsteuerung des Prozesses durchzuführen.

Die Erfindung löst diese Aufgabe durch ein Verfahren zur gleichmäßigen oder gesteuerten Bereitstellung von Rauchgas aus einer endotherm katalysierten Reaktion, wodurch sich durch dieses Rauchgas über einen Dampferzeuger ein in der Menge möglichst großer Dampfstrom produzieren lässt, wobei sich im Ausgang des Rauchgaskanals mindestens ein Hilfsbrenner befindet, durch den ein Rauchgasstrom erzeugt wird, der nicht mit den zu beheizenden Reaktionsrohren in Kontakt kommt und durch den der Rauchgasstrom an den Wärmeaustauschflächen des Dampferzeugers während der Regenerierung in der Menge erhöht wird. Die Erfindung löst diese Aufgabe auch durch eine Vorrichtung, die durch einen oder mehrere Hilfsbrenner am Eingang des Rauchgaskanals eines Reaktors zur Durchführung einer endotherm katalysierten Reaktion gebildet wird, wobei die Hilfsbrenner im Rauchgasstrom hinter den Reaktionsrohren angeordnet sind. Die Vorrichtung beinhaltet auch eine Einrichtung, mit der die Hilfsbrenner überwacht und gesteuert werden können.

Mittels der hier vorliegenden Erfindung kann die Menge des zugeführten Dampfes reduziert werden. Dabei entstehen keine zusätzlichen Kosten für Wärmetauschereinrichtungen; da die für den Normalbetrieb dimensionierten Apparate und Einbauten genutzt werden. Die Hilfsbrenner, die im Wesentlichen für den Regenerationsbetrieb benötigt werden, sind verhältnismäßig preiswert.

Typische endotherm katalysierte Verfahren, für die die Erfindung zur Anwendung kommt, sind Dehydrierungen von Alkanen. In jedem Fall sind dies Reaktionen, die in mit Katalysator befüllbaren Reaktionsrohren durchgeführt werden, wobei die Reaktionsrohre in einem beheizbaren Reaktionsraum sitzen und dieser Reaktionsraum durch Brenner mit einem Heizgas beheizt wird. Ein typisches Verfahren zur Dehydrierung von Alkanen, in der die Erfindung anwendbar ist, beschreibt die WO 2004/039920 A2. Darin wird auch ein Reaktor beschrieben, durch den in einer separaten Prozessstufe der bei Alkandehydrierung entstehende Wasserstoff verbrannt wird.

Beansprucht wird insbesondere ein Verfahren zur Regenerierung eines Festbettkatalysators mit einer zeitlich konstanten Erzeugung von Dampf aus der Beheizung des Reaktors, wobei
- ein Festbettkatalysator in einem oder mehreren Reaktionsrohren untergebracht ist, in welchem eine endotherme Reaktion unter Durchströmung eines zu reagierenden Gasgemisches durchgeführt wird, und
- das oder die Reaktionsrohre von außen durch Verbrennung eines Heizgases in einem Heizraum beheizt werden, durch welchen die Reaktionsrohre zur Durchführung der endothermen Reaktion geführt werden, und
- die Reaktion in dem oder den Reaktionsrohren in einem begrenzten Zeitraum zyklisch durchgeführt wird, wobei der nicht für die Reaktion genutzte Zeitraum für die Regenerierung des Katalysators durch Überleitung eines sauerstoff- oder wasserdampfhaltigen Gases oder einem Gemisch dieser Gase genutzt wird, und
- der durch die Beheizung der Reaktionsrohre entstehende Rauchgasstrom aus dem Heizraum ausgeführt wird und dieser bei der Ausführung über einen Dampferzeuger zur Erzeugung von Dampf genutzt wird,
   und das dadurch gekennzeichnet ist, dass
- sich im Ausgang des Rauchgaskanals mindestens ein Hilfsbrenner befindet, durch den ein Rauchgasstrom erzeugt wird, der nicht mit den zu beheizenden Reaktionsrohren in Kontakt kommt und durch den der Rauchgasstrom an den Wärmeaustauschflächen des Dampferzeugers während der Regenerierung in der Menge erhöht wird.

Durch die Erhöhung der Menge an Rauchgas im Rauchgaskanal können die Wärmeaustauschflächen im Rauchgaskanal während der Regenerationsphase des Katalysators besser genutzt werden. Dadurch lässt sich die Menge an produziertem Dampf über die Gesamtzeitdauer des Prozesses nahezu konstant halten. Die Hilfsbrenner sind hierzu mit einer Regelungseinrichtung ausgestattet, durch die sich die Verbrennungsgasmenge steuern lässt. Dies kann durch eine Regelung der Hilfsbrenner in Abhängigkeit von der Rauchgastemperatur im Rauchgasstrom hinter den Hilfsbrennern erfolgen. Die Regelung steuert dabei den Zufluss an Heizgas oder Verbrennungsluft in die Hilfsbrenner.

Um die Erzeugung von Dampf während der Regeneration weiter zu erhöhen, werden die Hilfsbrenner vorteilhaft so eingesetzt, dass die Temperatur des Rauchgasstroms am Eingang des Rauchgaskanals an den Wärmeaustauschflächen des Dampferzeugers erhöht wird. Die Steuerung der Temperatur im Rauchgaskanal und an den Wärmeaustauschflächen kann dabei über die zugeführte Menge an Frischluft in den Rauchgaskanal gesteuert werden, wenn dies benötigt wird.

Die Hilfsbrenner werden vorteilhaft mit einer Regelungseinrichtung ausgestattet, so dass die Heizgaszufuhr und damit die Produktion von Rauchgas geregelt werden kann. Die Regelungseinrichtung wird dabei über einen Temperatursensor gesteuert, der sich in der Nähe der Wärmeaustauschflächen des Dampferzeugers befindet, so dass die Hilfsbrenner in Abhängigkeit von der Temperatur im Rauchgaskanal gesteuert werden können. In einer einfacheren Ausführungsart kann auch eine manuelle Steuerung der Hilfsbrenner vorgenommen werden.

Die Regelungseinrichtung der Hilfsbrenner kann auch über die Menge des produzierten Dampfes gesteuert werden. In diesem Fall sitzt an einer geeigneten Stelle des Dampferzeugers eine Dampfmengenmesseinrichtung für die Erzeugungsmenge des Dampfes, so dass die Hilfsbrenner in Abhängigkeit von der Menge des produzierten Dampfes gesteuert werden können.

Prozesse, die für das erfindungsgemäße Verfahren in Frage kommen, sind insbesondere Alkandehydrierungen, durch die ein Alkan unter Abgabe von Wasserstoff in ein Alken umgewandelt wird. Dies kann als einziger Prozess durchgeführt werden. Häufig werden damit aber auch Alkandehydrierungen betrieben, durch die ein Alkan unter Abgabe von Wasserstoff in ein Alken umgewandelt wird und der Wasserstoff in einem nachfolgenden separaten Prozessschritt oxidiert wird, wobei es zu einer weiteren Dehydrierung von bisher nicht umgesetztem Alkan kommt. Die Hilfsbrenner können dann in einem oder in mehreren Reaktoren installiert werden. Dadurch wird der gesamte endotherme Prozess mit der Regenerierung des Katalysators unterstützt.

Beansprucht wird auch eine Vorrichtung, mit der sich das erfindungsgemäße Verfahren ausführen lässt. Beansprucht wird insbesondere eine Vorrichtung zur gleichmäßigen Dampferzeugung aus der Abwärme einer Alkandehydrierung, umfassend
- einen Reaktor mit Heizraum zur Durchführung einer endothermen Reaktion und darin enthaltene Reaktionsrohre, die mit einem Katalysator befüllbar sind, einen Eingang für das Reaktionsgas und einen Ausgang für das Produktgas in den Reaktionsrohren, einen oder mehrere Hauptbrenner, die nicht mit dem Katalysator oder dem Reaktionsgas in Kontakt kommen und die Reaktionsrohre in dem Heizraum von außen beheizen, und am Ende des Heizraumes einen Rauchgaskanal mit Ausgang für das Rauchgas, und im Rauchgaskanal einen oder mehrere Dampferzeuger,
   und die dadurch gekennzeichnet ist, dass
- am Ausgang in den Rauchgaskanal hinter den Reaktionsrohren und vor dem Durchströmen des oder der Dampferzeuger ein oder mehrere Hilfsbrenner sitzen.

Reaktoren zur Durchführung von endothermen Prozessen, die Hilfsbrenner zur Überbrückung des Anfahrprozesses besitzen, sind im Stand der Technik bekannt. Die US 2003/0101651 A1 beschreibt eine Vorrichtung für eine endotherm katalysierte Reaktion, durch die ein kohlenwasserstoffhaltiges Gas durch mit einem Katalysator befüllbare Rohre geleitet wird, die von außen beheizt werden, wobei das Reaktionsgas durch Konvektion im Gegenstrom erhitzt wird. Durch die Reaktionsanordnung kann die Größe des Reaktors erheblich reduziert und die gesamte Anordnung transportabel gebaut werden. Die Vorrichtung beschreibt Hilfsbrenner, die zum Starten der Reaktion genutzt werden, indem diese in den Heizraum zur Verbrennung des Heizgases gegeben werden. Eine Regeneration des Katalysators oder eine Unterbrechung des Heizprozesses wird nicht beschrieben. Auch wird keine Steuerungseinrichtung für die Hilfsbrenner genannt.

In den häufigsten Ausführungsformen der Erfindung sind die Hilfsbrenner mit Regelungseinrichtungen versehen, durch die sich der Brenner in der Leistung regeln lässt. Geeignete Regelungseinrichtungen sind beispielsweise Ventile, Schieber, Klappen oder Spindeln, mit denen sich die Zufuhr von Brenngas in die Hilfsbrenner regeln lässt. Die Regelungseinrichtung kann auch eine Regelung für die Zufuhr von Verbrennungsluft in die Hilfsbrenner beinhalten.

Die Hilfsbrenner können auch in Abhängigkeit von Messparametern im Ausgang des Rauchgaskanals gesteuert werden. Dies sind im Zusammenhang mit den entsprechenden Verfahrensansprüchen insbesondere Messfühler für die Messung der Menge an Verbrennungsgas oder für die Temperatur des Verbrennungsgases. Diese sitzen zur Erfüllung dieses Zweckes im Ausgang des Rauchgaskanals. Wird eine Regelung des Hilfsbrennereinsatzes in Abhängigkeit von der Verbrennungsgasmenge gewünscht, so sind die Rauchgaskanäle mit einer Messeinrichtung ausgestattet, durch die die Verbrennungsgasmenge des Rauchgases im Rauchgaskanal gemessen werden kann und sich davon abhängig der Hilfsbrenner regeln lässt.

Wird eine Regelung des Hilfsbrennereinsatzes in Abhängigkeit von der Temperatur im Rauchgasstrom gewünscht, so sind die Rauchgaskanäle mit einer Messeinrichtung ausgestattet, durch die die Temperatur des Rauchgases im Rauchgaskanal gemessen werden kann und durch die sich davon abhängig der Hilfsbrenner regeln lässt. Wird eine Vergleichsmessung im Vergleich zur Gesamtmenge oder -temperatur des Rauchgases benötigt, so können entsprechende Messfühler auch im Rauchgaskanal selbst oder an den Wärmeaustauschflächen des Dampferzeugers sitzen. In einer weiteren Ausführungsform kommen auch Lambda-Sonden zur Messung des Sauerstoffgehaltes im Rauchgaskanal in Betracht, wenn die Steuerung der Hilfsbrenner über den Sauerstoffgehalt im Rauchgaskanal erfolgt.

Der Reaktor zur Einbringung der erfindungsgemäßen Vorrichtung ist typischerweise so aufgebaut, wie dies im Stand der Technik üblich ist. Zur Ausführung der Erfindung sind dies ein Reaktor zur Durchführung einer endothermen Reaktion, darin enthaltene Reaktionsrohre, die mit einem Katalysator befüllbar sind, Brenner, die nicht mit dem Katalysator oder dem Reaktionsgas in Kontakt kommen und die Reaktionsrohre von außen beheizen, einen Eingang für das Reaktionsgas und einen Ausgang für das Produktgas in den Reaktionsrohren, einen Eingang für das Heizgas und einen Rauchgaskanal, und einen Dampferzeuger mit Wärmeaustauschflächen im oder hinter dem Ausgang des Rauchgaskanals. Die Hauptbrenner und die erfindungsgemäßen Hilfsbrenner können im Heizraum oder im Rauchgaskanal an beliebiger Stelle angebracht sein. Dies gilt auch für die zu beheizenden Wärmeaustauschflächen. Die Brenner, Reaktionsrohre oder Dampferzeuger können einfach oder mehrfach vorhanden sein. Die Hilfsbrenner sind in jedem Fall so angeordnet, dass die daraus entweichenden Rauchgase nicht mit den Reaktionsrohren und dem darin enthaltenen Katalysator in Kontakt kommen.

Zur Steuerung und Regelung der Hilfsbrenner kommen insbesondere Vorrichtungen in Betracht, die im Stand der Technik zur Regelung von Brennern genutzt werden. Dies sind typischerweise Ventile, Schieber, Klappen oder Spindeln, mit denen sich die Zufuhr von Brenngas oder Verbrennungsluft in die Hilfsbrenner regeln lässt. Zur Messung von Steuerungsmesswerten kommen insbesondere Thermoelemente, Druckmessgeräte, Gasdurchflussmesser und Sauerstoffsonden in Betracht.

Zur Anwendung als Hilfsbrenner können Gasbrenner, Flüssigkeitsbrenner, Raketenbrenner oder Feststoff-Gebläsebrenner kommen. Dies richtet sich nach der Größe des Rauchgaskanals und der Wärmeaustauschflächen. Zu der erfindungsgemäßen Vorrichtung der Hilfsbrenner gehören auch geeignete Einrichtungen zur Zündung. Dies können beispielsweise elektrische oder elektronische Zünder, Pilotbrenner oder Feuersteine sein. Die Hilfsbrenner sind bevorzugt mit einer Regelungseinrichtung ausgestattet, durch die der oder die Hilfsbrenner in der Leistung geregelt werden. Diese kann beispielsweise so gestaltet sein, dass der Rauchgaskanal mit einer Messeinrichtung ausgestattet ist, durch die die Temperatur im Rauchgaskanal für das Rauchgas gemessen werden kann und davon abhängig der oder die Hilfsbrenner in der Leistung regelbar sind.

Zur weiteren Nutzung der Abwärme werden Dampferzeuger genutzt, die beliebig angeordnet sein können und in beliebiger Zahl vorhanden sein können. Typischerweise handelt es sich dabei um Dampferzeuger, die als indirekte Wärmetauscher mit Wärmeaustauschflächen angeordnet sind. Diese können beliebig gestaltet sein. Diese können auch Messeinrichtungen zur Messung der erzeugten Dampfmenge enthalten. Die Dampferzeuger, die mit dem oder den Hilfsbrennern beheizt werden, können mit einer Dampfmengenmesseinrichtung ausgestattet sein, so dass davon abhängig der oder die Hilfsbrenner in der Leistung regelbar sind. Ferner gehören zur erfindungsgemäßen Vorrichtung auch Hilfseinrichtungen, wozu Rohrieitungen gehören. Diese können in beliebiger Ausführungsform und -zahl vorhanden sein.

Die erfindungsgemäße Vorrichtung bietet den Vorteil einer zeitlich möglichst gleichmäßigen Erzeugung von Dampf aus der Abwärme einer Alkandehydrierung. Durch die Vorrichtung und das erfindungsgemäße Verfahren kann die Dampfproduktion aus der Abwärme der genannten Prozesse optimiert und daraus mechanische Energie gewonnen werden.

## Patentansprüche

1. Verfahren zur gleichmäßigen Dampferzeugung aus der Abwärme einer Alkandehydrierung, wobei
• ein Festbettkatalysator in einem oder mehreren Reaktionsrohren untergebracht ist, in welchem eine endotherme Reaktion unter Durchströmung eines zu reagierenden Gasgemisches durchgeführt wird, und
• das oder die Reaktionsrohre von außen durch Verbrennung eines Heizgases in einem Heizraum beheizt werden, durch welchen die Reaktionsrohre zur Durchführung der endothermen Reaktion geführt werden, und
• die Reaktion in dem oder den Reaktionsrohren in einem begrenzten Zeitraum zyklisch durchgeführt wird, wobei der nicht für die Reaktion genutzte Zeitraum für die Regenerierung des Katalysators durch Überleitung eines sauerstoff- oder wasserdampfhaltigen Gases oder einem Gemisch dieser Gase genutzt wird, und
• der durch die Beheizung der Reaktionsrohre entstehende Rauchgasstrom aus dem Heizraum ausgeführt wird und dieser bei der Ausführung über einen Dampferzeuger zur Erzeugung von Dampf genutzt wird,
**dadurch gekennzeichnet, dass**
• sich im Rauchgasfluss hinter den zu beheizenden Reaktionsrohren mindestens ein Hilfsbrenner befindet, durch den ein Rauchgasstrom erzeugt wird, der nicht mit den zu beheizenden Reaktionsrohren in Kontakt kommt und durch den der Rauchgasstrom an den Wärmeaustauschflächen des Dampferzeugers während der Regenerierung in der Menge erhöht wird.

2. Verfahren zur gleichmäßigen Dampferzeugung aus der Abwärme einer Alkandehydrierung nach Anspruch 1, **dadurch gekennzeichnet, dass** durch den oder die Hilfsbrenner der Rauchgasstrom an den Wärmeaustauschflächen des Dampferzeugers während der Regenerierung in der Temperatur erhöht wird.

3. Verfahren zur gleichmäßigen Dampferzeugung aus der Abwärme einer Alkandehydrierung nach einem der Ansprüche 1 bis 2, **dadurch gekennzeichnet, dass** die Hilfsbrenner mit einer Regelungseinrichtung ausgestattet sind und der Rauchgaskanal, der mit dem Hilfsbrenner beheizt wird, mit einer Temperaturmesseinrichtung ausgestattet ist, so dass die Hilfsbrenner in Abhängigkeit von der Temperatur im Rauchgaskanal geregelt werden können.

4. Verfahren zur gleichmäßigen Dampferzeugung aus der Abwärme einer Alkandehydrierung nach einem der Ansprüche 1 bis 3, **dadurch gekennzeichnet, dass** die Hilfsbrenner mit einer Regelungseinrichtung ausgestattet sind und die Dampferzeuger, die mit dem Hilfsbrenner beheizt werden, mit einer Dampfmengenmesseinrichtung ausgestattet sind, so dass die Hilfsbrenner in Abhängigkeit von der Menge des produzierten Dampfes gesteuert werden können.

5. Verfahren zur gleichmäßigen Dampferzeugung aus der Abwärme einer Alkandehydrierung nach einem der Ansprüche 1 bis 4, **dadurch gekennzeichnet, dass** es sich bei dem endothermen Prozess mit dem zu regenerierenden Katalysator um eine Alkandehydrierung handelt, durch die ein Alkan unter Abgabe von Wasserstoff in ein Alken umgewandelt wird.

6. Verfahren zur gleichmäßigen Dampferzeugung aus der Abwärme einer Alkandehydrierung nach einem der Ansprüche 1 bis 5, **dadurch gekennzeichnet, dass** es sich bei dem endothermen Prozess mit dem zu regenerierenden Katalysator um eine Alkandehydrierung handelt, durch die ein Alkan unter Abgabe von Wasserstoff in ein Alken umgewandelt wird und der Wasserstoff in einem nachfolgenden separaten Prozessschritt oxidiert wird, wobei es zu einer weiteren Dehydrierung von bisher nicht umgesetztem Alkan kommt.

7. Vorrichtung zur gleichmäßigen Dampferzeugung aus der Abwärme einer Alkandehydrierung, umfassend
• einen Reaktor mit Heizraum zur Durchführung einer endothermen Reaktion und darin enthaltene Reaktionsrohre, die mit einem Katalysator befüllbar sind,
• einen Eingang für das Reaktionsgas und einen Ausgang für das Produktgas in den Reaktionsrohren,
• einen oder mehrere Hauptbrenner, die nicht mit dem Katalysator oder dem Reaktionsgas in Kontakt kommen und die Reaktionsrohre in dem Heizraum von außen beheizen,
• am Ende des Heizraumes einen Rauchgaskanal, und im Rauchgaskanal einen oder mehrere Dampferzeuger,
**dadurch gekennzeichnet, dass**
• im Ausgang des Rauchgaskanals im Rauchgasstrom hinter den zu beheizenden Reaktionsrohren und vor dem Durchströmen des oder der Dampferzeuger ein oder mehrere Hilfsbrenner sitzen.

8. Vorrichtung zur gleichmäßigen Dampferzeugung aus der Abwärme einer Alkandehydrierung oder einer Dampfreformierung nach Anspruch 7, **dadurch gekennzeichnet, dass** der oder die Hilfsbrenner mit einer geeigneten Zündvorrichtung ausgestattet sind.

9. Vorrichtung zur gleichmäßigen Dampferzeugung aus der Abwärme einer Alkandehydrierung oder einer Dampfreformierung nach einem der Ansprüche 7 oder 8, **dadurch gekennzeichnet, dass** der oder die Hilfsbrenner mit einer Regelungseinrichtung ausgestattet sind, durch die der oder die Hilfsbrenner in der Leistung geregelt werden.

10. Vorrichtung zur gleichmäßigen Dampferzeugung aus der Abwärme einer Alkandehydrierung oder einer Dampfreformierung nach einem der Ansprüche 7 bis 9, **dadurch gekennzeichnet, dass** der Rauchgaskanal mit einer Messeinrichtung ausgestattet ist, durch die die Temperatur im Rauchgaskanal für das Rauchgas gemessen werden kann, so dass davon abhängig der oder die Hilfsbrenner in der Leistung regelbar sind.

11. Vorrichtung zur gleichmäßigen Dampferzeugung aus der Abwärme einer Alkandehydrierung nach einem der Ansprüche 7 bis 9, **dadurch gekennzeichnet, dass** die Dampferzeuger, die mit dem oder den Hilfsbrennern beheizt sind, mit einer Dampfmengenmesseinrichtung ausgestattet sind, so dass davon abhängig der oder die Hilfsbrenner in der Leistung regelbar sind.

## Claims

1. A method of constant steam generation from the waste heat of an alkane dehydrogenation,
wherein
- a fixed-bed catalyst is accommodated in one or more reactor tubes, in which catalyst an endothermic reaction is performed while a mixture of gases to be reacted flows through,and
- said reactor tube or tubes are heated from the outside by burning a heating gas in a heating chamber through which the reactor tubes are passed to perform the endothermic reaction, and
- the reaction in the reactor tube or tubes is performed cyclically in a limited time period, the period not used for reaction being used for regenerating the catalyst by passing thereover a gas containing oxygen or water vapor, or a mixture of these gases, and
- the flue gas stream resulting from heating the reactor tubes is discharged from the heating chamber and is used, upon discharge, to generate steam via a steam generator,
**characterized in that**
- located in the flue gas stream downstream from the reactor tubes to be heated there is at least one auxiliary burner by which a flue gas stream is generated which does not get into contact with the reactor tubes to be heated, and by which the flue gas stream at the heat exchanging surfaces of the steam generator is increased in amount during regeneration.

2. The method of constant steam generation from the waste heat of an alkane dehydrogenation of claim 1, **characterized in that** the flue gas stream at the heat exchanging surfaces or the steam generator is increased in temperature by the auxiliary burner or burners during regeneration.

3. The method of constant steam generation from the waste heat of an alkane dehydrogenation of one of claims 1 to 2, **characterized in that** the auxiliary burners are provided with a controlling device and that the flue gas channel heated by means of the auxiliary burner is provided with a temperature measuring device so that the auxiliary burners can be controlled depending on the temperature in the flue gas channel.

4. The method of constant steam generation from the waste heat of an alkane dehydrogenation of one of claims 1 to 3, **characterized in that** the auxiliary burners arc provided with a controlling device, and the steam generators heated by means of the auxiliary burner are provided with a measuring device for the amount of steam, so that the auxiliary burners can be controlled depending on the amount of steam produced.

5. The method of constant steam generation from the waste heat of an alkane dehydrogenation of one of claims 1 to 4, **characterized in that** the endothermic process with the catalyst to be regenerated is an alkane dehydrogenation by which an alkane is converted to an alkene while releasing hydrogen.

6. The method of constant steam generation from the waste heat of an alkane dehydrogenation of one of claims 1 to 5, **characterized in that** the endothermic process with the catalyst to be regenerated is an alkane dehydrogenation by which an alkane is converted to an alkene while releasing hydrogen, and the hydrogen is oxidized in a subsequent separate process step, during which there is further dehydrogenation of alkane which has not been converted yet.

7. A device for constant steam generation from the waste heat of an alkane dehydrogenation, comprising
- a reactor having a heating chamber for performing an endothermic reaction, and reactor tubes contained therein which can be filled with a catalyst,
- an inlet for the reaction gas and an outlet for the product gas in the reactor tubes,
- one or more main burners which do not get into contact with the catalyst or the reaction gas and which heat the reactor tubes in the heating chamber from the outside,
- a flue gas channel at the end of the heating chamber, and one or more steam generators in the flue gas channel,
**characterized in that**
- there are one or more auxiliary burners located in the outlet of the flue gas channel in the flue gas stream downstream from the reactor tubes to be heated and upstream from the flow through the steam generator or generators.

8. The device for constant steam generation from the waste heat of an alkane dehydrogenation or steam reformation of claim 7, **characterized in that** the auxiliary burner or burners are provided with a suitable ignition device.

9. The device for constant steam generation from the waste heat of an alkane dehydrogenation or steam reformation of one of claims 7 or 8, **characterized in that** the auxiliary burner or burners are provided with a controlling device by which the auxiliary burner or burners are controlled in their output.

10. The device for constant steam generation from the waste heat of an alkane dehydrogenation or steam reformation of one of claims 7 to 9, **characterized in that** the flue gas channel is provided with a measuring device by which the temperature can be measured in the flue gas channel for the flue gas, so that depending thereon the auxiliary burner or burners can be controlled in their output.

11. The device for constant steam generation from the waste heat of an alkane dehydrogenation of one of claims 7 to 9, **characterized in that** the steam generators heated by means of the auxiliary burner or burners are provided with a measuring device for the amount of steam, so that depending thereon the auxiliary burner or burners can be controlled in their output.

## Revendications

1. Procédé destiné à la génération uniforme de vapeur à partir de la chaleur dissipée d'une déshydrogénation d'alcanes, sachant que
• un catalyseur à lit fixe est logé dans un ou plusieurs tubes-laboratoires, dans lequel une réaction endotherme est exécutée avec la traversée d'un mélange gazeux à réagir, et
• le ou les tubes-laboratoires sont chauffés par l'extérieur par combustion d'un gaz de chauffage dans une chambre de chauffe, à travers laquelle les tubes-laboratoires sont conduits pour l'exécution de la réaction endotherme, et
• la réaction dans le ou les tubes-laboratoires est exécutée cycliquement dans une période limitée, sachant que la période non utilisée pour la réaction est utilisée pour la régénération du catalyseur par le passage d'un gaz contenant de l'oxygène ou de la vapeur d'eau ou d'un mélange de ces gaz, et
• le flux de gaz de fumées produit par le chauffage des tubes-laboratoires est conduit hors de la chambre de chauffe et celui-ci est utilisé lors du guidage au-dehors pour la génération de vapeur via un générateur de vapeur,
**caractérisé en ce que**
• dans le flux de gaz de fumées, en aval des tubes-laboratoires à chauffer se trouve au moins un brûleur auxiliaire, par lequel un flux de gaz de fumées est généré, qui n'entre pas en contact avec les tubes-laboratoires à chauffer et par lequel le flux de gaz de fumées est augmenté en quantité sur les surfaces d'échange thermique du générateur de vapeur pendant la régénération.

2. Procédé destiné à la génération uniforme de vapeur à partir de la chaleur dissipée d'une déshydrogénation d'alcanes selon la revendication 1, **caractérisé en ce que** par le ou les brûleurs auxiliaires, le flux de gaz de fumées est augmenté en température sur les surfaces d'échange thermique du générateur de vapeur pendant la régénération.

3. Procédé destiné à la génération uniforme de vapeur à partir de la chaleur dissipée d'une déshydrogénation d'alcanes selon une quelconque des revendications 1 à 2, **caractérisé en ce que** les brûleurs auxiliaires sont équipés d'un dispositif de régulation et le rampant qui est chauffé avec le brûleur auxiliaire, est équipé d'un système de mesure de la température, de sorte que les brûleurs auxiliaires peuvent être réglés en fonction de la température dans le rampant.

4. Procédé destiné à la génération uniforme de vapeur à partir de la chaleur dissipée d'une déshydrogénation d'alcanes selon une quelconque des revendications 1 à 3, **caractérisé en ce que** les brûleurs auxiliaires sont équipés d'un dispositif de régulation et les générateurs de vapeur qui sont chauffés avec le brûleur auxiliaire, sont équipés d'un système de mesure de la quantité de vapeur, de sorte que les brûleurs auxiliaires peuvent être commandés en fonction de la quantité de la vapeur produite.

5. Procédé destiné à la génération uniforme de vapeur à partir de la chaleur dissipée d'une déshydrogénation d'alcanes selon une quelconque des revendications 1 à 4, **caractérisé en ce que**, pour le processus endotherme avec le catalyseur à régénérer, il s'agit d'une déshydrogénation d'alcanes, par laquelle un alcane est transformé en alcène avec dégagement d'hydrogène.

6. Procédé destiné à la génération uniforme de vapeur à partir de la chaleur dissipée d'une déshydrogénation d'alcanes selon une quelconque des revendications 1 à 5, **caractérisé en ce que** pour le processus endotherme avec le catalyseur à régénérer, il s'agit d'une déshydrogénation d'alcanes, par laquelle un alcane est transformé en alcène avec dégagement d'hydrogène et l'hydrogène est oxydé dans une étape de processus séparée suivante, sachant qu'une autre déshydrogénation se produit de l'alcane non transformé jusqu'à présent.

7. Dispositif destiné à la génération uniforme de vapeur à partir de la chaleur dissipée d'une déshydrogénation d'alcanes, comprenant
• un réacteur avec chambre de chauffe destiné à l'exécution d'une réaction endotherme et des tubes-laboratoires contenus dans celui-ci, qui sont remplissables d'un catalyseur,
• une entrée pour le gaz de réaction et une sortie pour le gaz de production dans les tubes-laboratoires,
• un ou plusieurs brûleurs principaux qui n'entrent pas en contact avec le catalyseur ou le gaz de réaction et chauffent par l'extérieur les tubes-laboratoires dans la chambre de chauffe,
• au bout de la chambre de chauffe, un rampant, et dans le rampant, un ou plusieurs générateurs de vapeur,
**caractérisé en ce que**
• à la sortie du rampant dans le flux de gaz de fumées, un ou plusieurs brûleurs auxiliaires sont placés en aval des tubes-laboratoires à chauffer et en amont de la traversée du ou des générateurs de vapeur.

8. Dispositif destiné à la génération uniforme de vapeur à partir de la chaleur dissipée d'une déshydrogénation d'alcanes ou d'une épuration à la vapeur selon la revendication 7, **caractérisé en ce que** le ou les brûleurs auxiliaires sont équipés d'un dispositif d'allumage adéquat.

9. Dispositif destiné à la génération uniforme de vapeur à partir de la chaleur dissipée d'une déshydrogénation d'alcanes ou d'une épuration à la vapeur selon une quelconque des revendications 7 ou 8, **caractérisé en ce que** le ou les brûleurs auxiliaires sont équipés d'un dispositif de régulation, par lequel le ou les brûleurs auxiliaires sont régulés en puissance.

10. Dispositif destiné à la génération uniforme de vapeur à partir de la chaleur dissipée d'une déshydrogénation d'alcanes ou d'une épuration à la vapeur selon une quelconque des revendications 7 à 9, **caractérisé en ce que** le rampant est équipé d'un système de mesure, par lequel la température peut être mesurée dans le rampant pour le gaz de fumée, de sorte qu'en fonction de celui-ci, le ou les brûleurs auxiliaires sont réglables en puissance.

11. Dispositif destiné à la génération uniforme de vapeur à partir de la chaleur dissipée d'une déshydrogénation d'alcanes selon une quelconque des revendications 7 à 9, **caractérisé en ce que** les générateurs de vapeur, qui sont chauffés avec le ou les brûleurs auxiliaires, sont équipés d'un système de mesure de la quantité de vapcur, de sorte qu'en fonction de celui-ci, le ou les brûleurs auxiliaires sont réglables en puissance.
